# EUROPÄISCHE PATENTSCHRIFT

(11) **EP 1 110 511 B1**
(45) Veröffentlichungstag und Bekanntmachung des Hinweises auf die Patenterteilung: **17.09.2014**
(21) Anmeldenummer: 00811131.2
(22) Anmeldetag: 28.11.2000
(51) Int. Cl.: A61B 17/88, B25B 23/142

(54) **Medizinaltechnische Werkzeughaltevorrichtung mit Drehmomentbegrenzung**
Medical tool holder with torque limitation
Porte-outil médical avec limitation de couple

(30) Priorität: 20.12.1999 EP 99811172
(43) Veröffentlichungstag der Anmeldung: 27.06.2001
(73) Patentinhaber: Zimmer GmbH, 8404 Winterthur (CH)
(72) Erfinder: Casutt, Simon, 9200 Gossau (CH)
(74) Vertreter: Manitz, Finsterwald & Partner GbR

(56) Entgegenhaltungen:
- US-A- 5 158 458
- US-A- 5 347 894
- US-A- 5 368 480

## Beschreibung

Die Erfindung betrifft eine medizinaltechnische Werkzeughaltevorrichtung mit Drehmomentbegrenzung gemäss dem Oberbegriff von Anspruch 1.

Die Erfindung betrifft weiter einen Bausatz für eine medizinaltechnische Werkzeughaltevorrichtung mit Drehmomentbegrenzung gemäss dem Oberbegriff von Anspruch 14.

Aus der Mechanik ist eine Vielzahl von drehmomentübertragenden Kupplungen bekannt, welche nur ein begrenztes Drehmoment übertragen. Derartige Kupplungen werden auch als Überlastkupplungen bezeichnet.

Nachteilig an derartigen Kupplungen ist die Tatsache, dass sie für medizinaltechnische Anwendungen, beispielsweise eingebaut in Instrumente für chirurgische Eingriffe am Menschen, nicht geeignet sind, da das maximal erzielbare Drehmoment relativ ungenau ist, und das maximal erzielbare Drehmoment längerfristig Änderungen unterworfen ist.

US 5,368,480 offenbart eine zahnmedizinische Ratsche, d.h. ein Schraubwerkzeug mit Drehmomentbegrenzung, mit einem Haltegriff und einem Ratschenkopf. Der Ratschenkopf umfasst eine Buchse, in die ein Werkzeugadapter für ein Werkzeug einsetzbar ist. Der Werkzeugadapter ist mit sechs radial nach außen orientierten, in Umfangsrichtung gleichverteilten Scherkörpern in Form von Nocken versehen. Die Buchse ist mit einem radial nach innen orientierten Schermittel versehen, das jeweils zwischen zwei benachbarte Nocken eingreift. Wird der Haltegriff gedreht, wirkt ein entsprechendes Drehmoment auf das Schermittel. Bis zu einem bestimmten Drehmoment kann das einwirkende Drehmoment über die Nocken auf den Werkzeugadapter und damit das Werkzeug übertragen werden. Wird das bestimmte Drehmoment jedoch überschritten, wird der Nocken, an dem das Schermittel zu diesem Zeitpunkt anliegt, derart plastisch verformt, dass der Eingriff zwischen Schermittel und dem jeweiligen Nocken überwunden wird.

Es ist Aufgabe der vorliegenden Erfindung eine medizinaltechnische Werkzeughaltevorrichtung mit Drehmomentbegrenzung zu schaffen, welche ein relativ genaues maximales Drehmoment aufweist, wobei das maximale Drehmoment auch längerfristig, beispielsweise nach mehrmaliger Wasserdampfsterilisation, reproduzierbar ist.

Diese Aufgabe wird gelöst mit einer medizinaltechnischen Werkzeughaltevorrichtung mit Drehmomentbegrenzung aufweisend die Merkmale von Anspruch 1. Die Unteransprüche 2 bis 12 betreffen weitere, vorteilhafte Ausgestaltungen von medizinaltechnischen Werkzeughaltevorrichtungen mit Drehmomentbegrenzung.

Die Aufgabe wird insbesondere gelöst mit einer medizinaltechnischen Werkzeughaltevorrichtung mit Drehmomentbegrenzung, umfassend eine Aufnahmevorrichtung für zumindest einen Scherkörper sowie ein rotierbar mit der Aufnahmevorrichtung verbundenes Schermittel, welches derart bezüglich einem sich in der Aufnahmevorrichtung befindlichen Scherkörper angeordnet ist, dass das Schermittel am Scherkörper anliegt, zumindest wenn ein Drehmoment auf das Schermittel wirkt, wobei das Schermittel und der Scherkörper derart gegenseitig angepasst ausgestaltet und angeordnet sind, dass beim Überschreiten eines bestimmten Drehmomentes das Schermittel den Scherkörper zumindest teilweise durchtrennt.

Die erfindungsgemässe medizinaltechnische Werkzeughaltevorrichtung ist in einer bevorzugten Ausführungsform auf der einen Seite mit einem Handgriff und auf der anderen Seite mit einem Werkzeug verbunden und bildet derart ein medizinaltechnisches, beispielsweise ein chirurgisches Instrument. In einer bevorzugten Ausgestaltung ist der Handgriff länglich ausgestaltet und definiert dabei eine Längsachse, um welche das Instrument mitsamt dem Werkzeug gedreht wird, wobei die zwischen Handgriff und Werkzeug angeordnete Werkzeughaltevorrichtung das maximal zu übertragende Drehmoment begrenzt. Für grössere Momente, beispielsweise grösser als 5 Nm, kann der Handgriff auch als T-Griff ausgestaltet sein.

Die erfindungsgemässe medizinaltechnische Werkzeughaltevorrichtung weist den Vorteil auf, dass das maximal übertragbare Drehmoment durch die Verwendung eines Scherkörpers in Kombination mit einem Schermittel relativ genau definierbar ist. Der Scherkörper wird durch die Einwirkung des Schermittels zerstört und muss nach der Verwendung der Werkzeughaltevorrichtung ersetzt werden. Das Schermittel weist eine geringe Abnutzung auf und kann gegebenenfalls ebenfalls ersetzt werden. Die erfindungsgemässe medizinaltechnische Werkzeughaltevorrichtung weist daher den Vorteil auf, dass das maximal übertragbare Drehmoment auch über einen relativ grossen Zeitraum vom beispielsweise einem Jahr oder sogar mehreren Jahren relativ genau reproduzierbar ist. Zudem ist kein Kalibrieren der Werkzeughaltevorrichtung erforderlich.

Spätestens beim Drehen des Handgriffes gelangt das Schermittel zur Anlage an den Scherkörper, wobei das Schermittel mit zunehmendem Drehmoment in den Scherkörper eindringt und beim Überschreiten eines bestimmten Drehmomentes diesen teilweise oder vollständig durchtrennt. Ein durchgetrennter Scherkörper ist zerstört und muss ersetzt werden. In einer vorteilhaften Ausgestaltung weist die erfindungsgemässe Werkzeughaltevorrichtung eine Aufnahmevorrichtung für eine Mehrzahl von Scherkörpern auf, beispielsweise eine Aufnahmevorrichtung für sechs Scherkörper, sodass mehrmals nacheinander beim Überschreiten des bestimmten Drehmomentes der Scherkörper durchtrennt wird, die Scherkörper jedoch erst dann auszuwechseln sind, wenn alle sich in der Werkzeughaltevorrichtung befindlichen Scherkörper zerstört sind.

Die erfindungsgemässe medizinaltechnische Werkzeughaltevorrichtung erlaubt beispielsweise eine Schraube wie eine Stiftschraube oder eine Knochenschraube während dem Implantieren mit einem genau voreingestellten Drehmoment anzuziehen.

Die erfindungsgemässe Werkzeughaltevorrichtung weist den Vorteil auf, dass je nach Erfordernis unterschiedlich grosse maximale Drehmomente voreinstellbar sind. Das maximale Drehmoment zum Durchtrennen eines Scherkörpers wird bestimmt durch:
- die Materialeigenschaften des Scherkörpers,
- die geometrische Ausgestaltung des Scherkörpers,
- die geometrische Ausgestaltung des Schermittels, wobei das Schermittel vorzugsweise eine Klinge aufweist, deren Geometrie in einer Vielzahl von Formgebungen ausgestaltbar ist,

- den wirksamen Hebelarm des Schermittels;
- den Grad der Durchtrennung des Scherkörpers, teilweise oder vollständig.

Durch eine geeignete Kombination dieser Parameter lassen sich eine Vielzahl unterschiedlicher und genau reproduzierbarer maximaler Drehmomente erzielen. In einer bevorzugten Ausgestaltung wird immer dasselbe Schermittel verwendet, wobei Scherkörper mit unterschiedlichen Materialeigenschaften und/oder unterschiedlichen geometrischen Ausgestaltungen verwendet werden, sodass das maximale Drehmoment abhängig vom jeweiligen Scherkörper beispielsweise 4 Nm, 6 Nm oder 10 Nm beträgt. In einer bevorzugten Ausgestaltung weisen diese Scherkörper eine farbliche Kodierung auf, wobei Scherkörper mit derselben Farbe dasselbe maximale Drehmoment ergeben. Die erfindungsgemässe Werkzeughaltevorrichtung weist somit den Vorteil auf, dass das maximal erzielbare Drehmoment unter Verwendung derselben Vorrichtung in weiten Grenzen variierbar ist, in dem je nach dem erforderlichen maximalen Drehmoment ein entsprechender Scherkörper in die Aufnahmevorrichtung eingelegt wird. Gerade beim Implantieren eines orthopädischen Implantates wie einer Hüftgelenkpfanne kann das maximal erlaubte Drehmoment beispielsweise von der Grösse des gewählten Implantates abhängen. Die erfindungsgemässe Werkzeughaltevorrichtung ermöglicht es einem Chirurgen oder einer Hilfskraft während einer Operation das maximal Drehmoment des Instrumentes einzustellen, indem die entsprechenden Scherkörper in die Aufnahmevorrichtung eingelegt werden. Ein Satz unterschiedlicher Scherkörper muss dazu in sterilisierter Form vorrätig sein.

Ein chirurgisches Instrument umfassend die erfindungsgemässe medizinaltechnische Werkzeughaltevorrichtung kann beispielsweise als ein Drehmomentbegrenzungs-Schraubenzieher ausgestaltet sein.

Die erfindungsgemässe medizinaltechnische Werkzeughaltevorrichtung ist in einer bevorzugten Ausgestaltung aus sterilisierbarem Material, insbesondere Metall hergestellt, sodass der Vorrichtung nach einer Operation die Scherkörper entnommen werden, die Vorrichtung sterilisiert wird, und danach für weitere Operationen verwendbar ist.

Die erfindungsgemässe Werkzeughaltevorrichtung kann für unterschiedlichste Auslösemomente zusammengestellt werden, ohne die äussere Geometrie der Werkzeughaltevorrichtung oder des Abscherstiftes zu ändern. Die erfindungsgemässe Werkzeughaltevorrichtung kann unter Berücksichtigung des jeweils erforderlichen Auslösemomentes modulartig zusammengestellt werden, sodass mit wenigen Änderungen, zum Beispiel einem Ersatz einer Einfachklinge durch eine Doppelklinge, oder durch die Verwendung einer spitz oder stumpf ausgebildeten Klinge, ein Instrument mit dem gewünschten Auslösemoment zur Verfügung steht.

Die Erfindung wird weiter an Hand mehrerer Ausführungsbeispiele im Detail beschrieben. Es zeigen:
- Fig. 1a: eine Seitenansicht einer medizinaltechnischen Werkzeughaltevorrichtung eingebaut in ein medizinaltechnisches Instrument;
- Fig. 1b: ein Querschnitt durch die Werkzeughaltevorrichtung gemäss Fig. 1a entlang der Schnittlinie A-A;
- Fig. 1c: ein Längsschnitt durch die Werkzeughaltevorrichtung gemäss Fig. 1b entlang der Schnittlinie B-B;
- Fig. 2a: eine Explosionsdarstellung der Werkzeughaltevorrichtung;
- Fig. 2b: eine Detailansicht des Verschlusses einer Werkzeughaltevorrichtung;
- Fig. 3a: eine Ansicht einer Welle umfassend ein Schermittel;
- Fig. 3b: eine Aufsicht der Welle gemäss Fig. 3a;
- Fig. 3c: eine Seitenansicht der Welle gemäss Fig. 3b sowie ein Schnitt durch das Schermittel entlang der Schnittlinie C-C;
- Fig. 3d,3e: ein Schnitt durch weitere Ausführungsbeispiele von Schermitteln;
- Fig. 4: eine Ansicht einer Aufnahmevorrichtung;
- Fig. 5: eine Darstellung des Zusammenhangs zwischen dem Winkel α der Klinge sowie dem maximal erreichbaren Drehmoment;
- Fig. 6: ein Querschnitt entlang der Schnittlinie A-A mit einer kürzer ausgestalteten Klinge;
- Fig. 7: ein Ring aus sechs einzelnen oder fest miteinander verbundenen Scherkörpern;
- Fig. 8a: eine weiteres Ausführungsbeispiel einer Welle umfassend ein Schermittel sowie Ausnehmungen für eine Arretierungsvorrichtung;
- Fig. 8b: ein Längsschnitt durch ein weiteres Ausführungsbeispiel einer Werkzeughaltevorrichtung mit einem Freilauf;
- Fig. 9a: eine Aufsicht auf eine Haltevorrichtung für Abscherstifte;
- Fig. 9b: ein Schnitt durch die Haltevorrichtung gemäss Fig. 9a entlang der Schnittlinie C-C;
- Fig. 9c: eine perspektivische Ansicht der Haltevorrichtung;
- Fig. 10: eine Seitenansicht eines Abscherstifts;
- Fig. 11: eine perspektivische Ansicht einer Haltevorrichtung mit eingesetzten Abscherstiften.

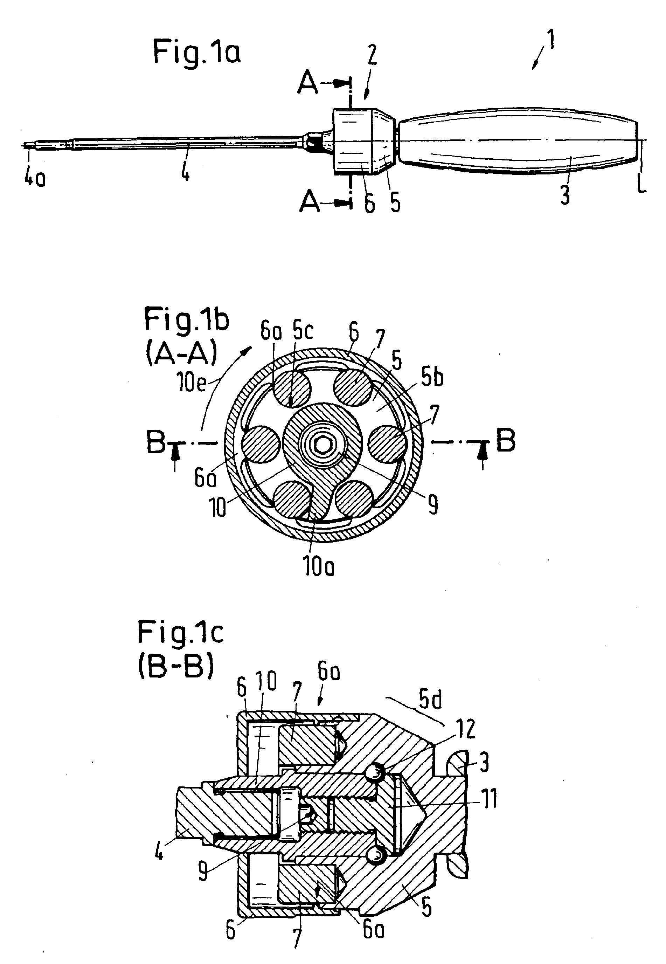

Fig. 1a zeigt die Seitenansicht eines chirugischen Instrumentes 1 umfassend eine medizinaltechnische Werkzeughaltevorrichtung 2 mit Drehmomentbegrenzung. Die Werkzeughaltevorrichtung 2 umfasst eine Aufnahmevorrichtung 5, welche zugleich ein erstes Teilgehäuse bildet, ein zweites Teilgehäuse 6 sowie eine in der Aufnahmevorrichtung 5 um eine gemeinsame Längsachse L drehbar gelagerte Welle 10, wobei die Welle 10 eine Aufnahme für ein Werkzeug 4 aufweist. An der Aufnahmevorrichtung 5 ist ein sich in Richtung der Längsachse L erstreckender Handgriff 3 befestigt. An der Welle 10 ist das lösbare, sich in Richtung der Längsachse L erstreckende Werkzeug 4 befestigt, an dessen Spitze 4a ein Sechskant zum Fassen einer Sechskantschraube angeordnet ist.

Fig. 1b zeigt im Querschnitt entlang der Schnittlinie A-A den inneren Aufbau der Werkzeughaltevorrichtung 2. Die Aufnahmevorrichtung 5 umfasst eine Haltevorrichtung 5b mit sechs über den Umfang regelmässig verteilt angeordneten Halteöffnungen 5c, in welchen je ein zylinderförmig ausgestalteter Scherkörper 7 angeordnet ist. Eine sich in Richtung der Längsachse L erstreckende Welle 10 weist ein bezüglich der Längsachse L radial vorstehendes Schermittel 10a auf. Die Welle 10 sowie das Schermittel 10a sind in der Aufnahmevorrichtung 5 um die Längsachse L drehbar gelagert. Das Schermittel 10a und die Scherkörper 7 sind derart gegenseitig angeordnet, dass das Schermittel 10a zwischen den Scherkörpern 7 liegt und bezüglich einer Drehbewegung in relativer Drehrichtung 10e behindert wird. Beim Überschreiten eines bestimmten Drehmomentes wird der Scherkörper 7, an welchem das Schermittel 10a anliegt, von diesem durchtrennt. Daher kann während dem Anziehen einer mit dem Instrument 1 eingeschraubten Schraube ein vorbestimmtes, maximales Drehmoment nicht überschritten werden. Das dargestellte Instrument 1 erlaubt maximal sechs Schrauben nacheinander mit einem bestimmten Drehmoment anzuziehen. Jedesmal wird beim erreichen des bestimmten Drehmomentes ein einzelner Scherkörper 7 durchtrennt, sodass nach sechs derartigen Vorgängen alle Scherkörper 7 durchtrennt sind und ersetzt werden müssen.

Der in Fig. 1c dargestellte Längsschnitt durch das Instrument 1 entlang der Linie B-B zeigt die Aufnahmevorrichtung 5 mit darin angeordneten Scherkörpern 7. Die Aufnahmevorrichtung 5 ist fest mit dem Handgriff 3 verbunden. In der Aufnahmevorrichtung 5 ist die Welle 10 und die Wellenarretierung 11 über ein Kugellager 12 drehbar gelagert, wobei die Welle 10 und die Wellenarretierung 11 von einer Konterschraube 9 zusammengehalten sind. Das Werkzeug 4 ist lösbar, beispielsweise steckbar oder schraubbar, mit der Welle 10 verbunden. Das Teilgehäuse 6 ist in Längsrichtung L verschiebbar und über eine Verbindung, beispielsweise einer Schnappverbindung, fest mit der Aufnahmevorrichtung 5 verbindbar.

Das Teilgehäuse 6 ist teilweise über eine Aussenfläche 5a der Aufnahmevorrichtung 5 gestülpt und umschliesst derart den von der Aufnahmevorrichtung 5, der Welle 10 sowie dem Teilgehäuse 6 gebildeten Innenraum. Dieser Innenraum ist vorzugsweise gegen Aussen abgedichtet, indem zwischen dem Teilgehäuse 6 und der Welle 10 beziehungsweise zwischen der Welle 10 und dem Werkzeug 4 eine Dichtung ausgebildet ist, sodass keine Flüssigkeit, insbesondere kein Wasser oder kein Blut in den Innenraum eindringen kann. Ebenso kann kein eventuell loser, sich im Innenraum befindlicher Gegenstand nach Aussen gelangen.

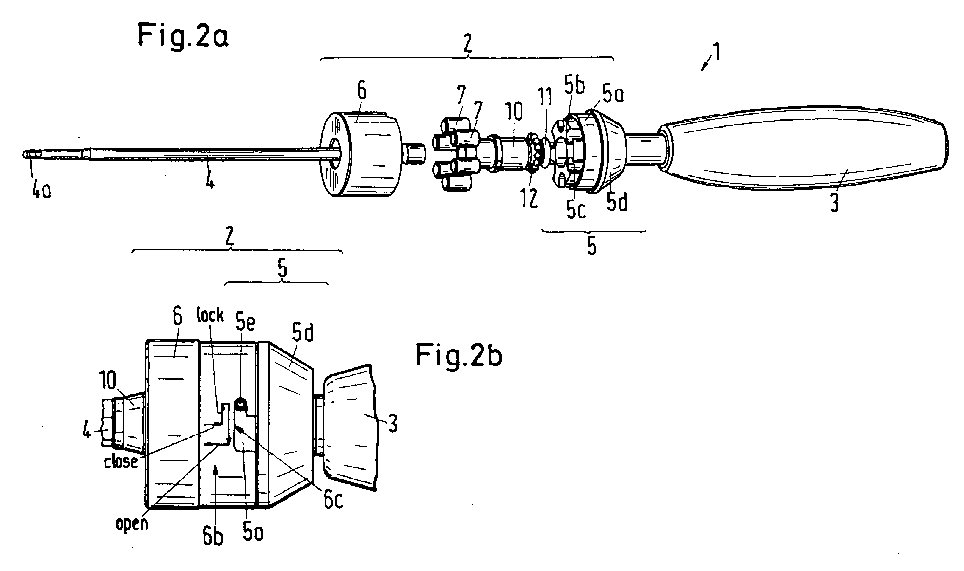

Fig. 2a zeigt in einer Explosionsdarstellung die einzelnen Bestandteile des Instrumentes 1 im Detail. Das Instrument 1 besteht aus dem Werkzeug 4, dem Haltegriff 3 sowie der diese beiden verbindenden medizinaltechnischen Werkzeughaltevorrichtung 2, welche die Aufnahmevorrichtung 5 mit Haltevorrichtung 5b, Welle 10 mit Wellenarretierung 11 und Kugellager 12, sowie ein Teilgehäuse 6 umfasst. Die als Verbrauchsmaterial konzipierten Scherkörper 7 sind in die Haltevorrichtung 5b einsetzbar.

Fig. 2b zeigt ein Ausführungsbeispiel einer Verschlussvorrichtung, um die Aufnahmevorrichtung 5 und das zweite Teilgehäuse 6 lösbar fest miteinander zu verbinden. Die Aufnahmevorrichtung 5 weist ein zylinderförmiges Teil 5a mit glatter Oberfläche auf, an welcher ein Stift 5e in bezüglich dem Teil 5a radialer Richtung vorstehend angeordnet ist. Das zweite Teilgehäuse 6 weist eine der Anordnung des Stiftes 5e angepasst ausgestaltete Ausnehmung 6c auf. Eine auf der Oberfläche des zweiten Teilgehäuses 6 angebrachte Markierung 6b stellt die erforderlichen Bewegungen dar, um um die Teile 5,6 zu verbinden oder zu lösen. Durch eine Bewegung in Richtung "close" wird das zweite Teilgehäuse 6 über das zylinderförmige Teil 5a geschoben, bis der Stift 5e an der Ausnehmung 6c anschlägt. Daraufhin wird die Aufnahmevorrichtung 5 in Richtung "lock" verdreht, bis der Stift 5e in der in Fig. 2b dargestellten Lage ist. In dieser Stellung sind die Aufnahmevorrichtung 5 und das zweite Teilgehäuse 6 fest verbunden und die Halteklingen 6a sind im Eingriff mit den Abscherstiften 7. Die Markierung "open" umfasst zwei zueinander senkrecht verlaufende Richtungen. Um die Verbindung zu lösen wird die Aufnahmevorrichtung 5 vorerst in Richtung "open" verdreht und danach das zweite Teilgehäuse 6 in Richtung "open" (senkrecht zur Drehrichtung) abgezogen. Aus Fig. 2b ist ersichtlich, dass schon ein geringes Verdrehen der Aufnahmevorrichtung 5 in Richtung "open" genügt, um den Stift 5e aus der Ausnehmung 6c zu entfernden. Je nach dem wie weit die Aufnahmevorrichtung 5 beim Öffnen in Richtung "open" verdreht wird, werden die Abscherstifte 7, bedingt durch die Stellung der Halteklingen 6a, beim Abziehen des zweiten Teilgehäuses 6 entweder gleichzeitig aus der Haltevorrichtung 5b, oder sie verbleiben in der Haltevorrichtung 5b.

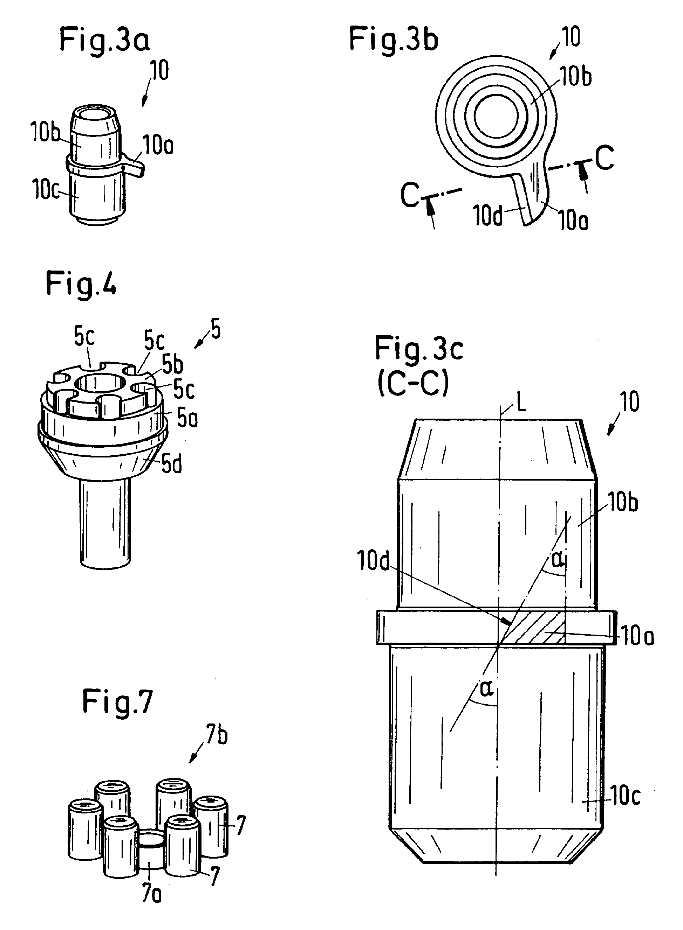

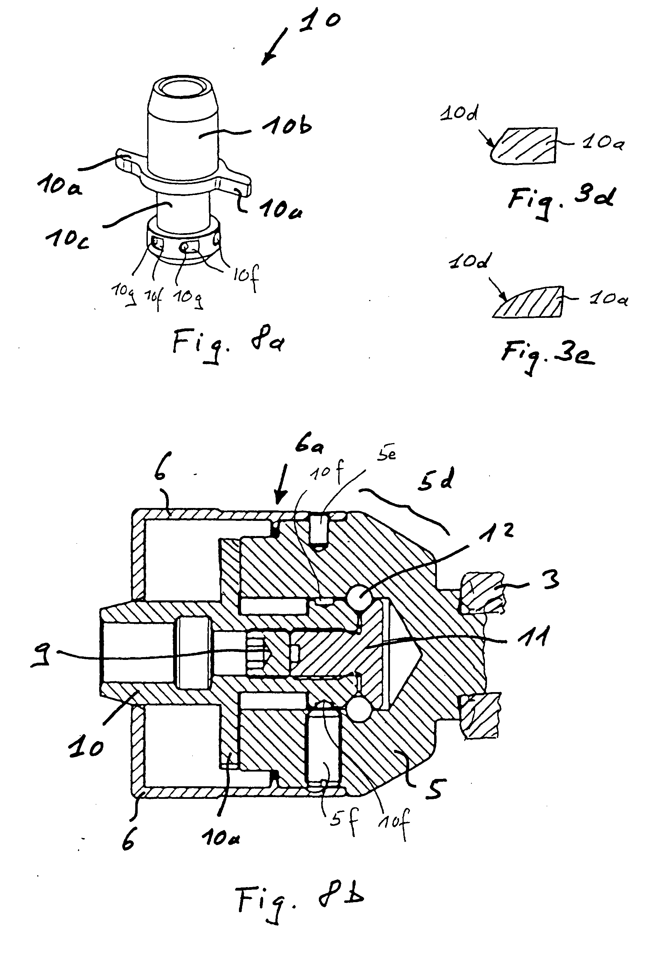

Fig. 3a zeigt die einstückig ausgebildete Welle 10 mit oberem Wellenteil 10b, unterem Wellenteil 10c sowie dem vorstehenden Schermittel 10a im Detail. Aus der Aufsicht gemäss Fig. 3b ist das obere Wellenteil 10b, das Schermittel 10a und dessen Klinge 10d ersichtlich. Die Seitenansicht gemäss Fig. 3c zeigt die in Längsrichtung L verlaufende Welle 10 mit oberem Wellenteil 10b und unterem Wellenteil 10c. Das Schermittel 10a ist entlang der Linie C-C geschnitten dargestellt und zeigt eine flächig verlaufende, bezüglich der Längsachse L um einen Winkel α geneigt verlaufende Klinge 10d. Die Klinge 10d kann neben einer ebenen Schneidfläche auch in einer Vielzahl anderer geometrischer Formen ausgestaltet sein, und beispielsweise, wie in einem Schnitt in den Figuren 3d und 3e dargestellt, eine teilweise gerundete oder elliptische Schneidfläche aufweisen.

Fig. 4 zeigt eine perspektivische Ansicht der Aufnahmevorrichtung 5 mit Aussengewinde 5a, Haltevorrichtung 5b mit Halteöffnungen 5c für die Scherkörper 7 sowie ein Aussengehäuse 5d. Die beispielsweise zylinderförmig ausgestalteten Scherkörper 7 bzw. Abscherstifte 7 können einzeln in die Aufnahmevorrichtung 5 eingesetzt werden. Es sind nur so viele Scherkörper 7 einzusetzen wie benötigt werden. Im dargestellten Ausführungsbeispiel gemäss der Figuren 1a, 1b, 1c werden die Scherkörper 7 in die Aufnahmevorrichtung 5 eingesetzt, danach das Teilgehäuse 6 in Längsrichtung L bewegt, bis dieses über die Fläche 5a gestülpt ist. Daraufhin wird das Teilgehäuse 6 leicht in Richtung 10e gedreht, bis das Teilgehäuse 6 in eine nicht dargestellte Rasteinrichtung einschnappt und dadurch fest in dessen Lage gehalten ist. Das Gehäuse 6 umfasst sechs über den Umfang verteilt angeordnete, radial nach innen vorstehende Halteklingen 6a, welche beim Drehen in Richtung 10e in die Scherkörper 7 eindringen und diese in ihrer Lage fixieren. Dadurch ist gewährleistet, dass sich die Scherkörper 7 während dem Gebrauch des Werkzeuges 1 nicht frei im Innenraum der Drehmomentbegrenzungsvorrichtung 2 bewegen. In einer vorteilhaften Ausgestaltung werden die Scherkörper 7 vom Schermittel 10a nur teilweise durchtrennt, sodass durch die Scherwirkung keine losen, insbesondere keine grösseren losen Teile erzeugt werden.

Fig. 7 zeigt ein weiteres Ausführungsbeispiel eines Abschermittels 7b, welches aus sechs über einen Haltering 7a fest miteinander verbundenen Scherkörpern 7 besteht. Das gesamte Abschermittel 7b ist mit einem Handgriff in die Aufnahmevorrichtung 5 einsetzbar. Das Abschermittel 7b könnte auch derart ausgestaltet sein, dass die Scherkörper 7 nur geringfügig oder gar nicht mit dem Haltering 7a verbunden sind.

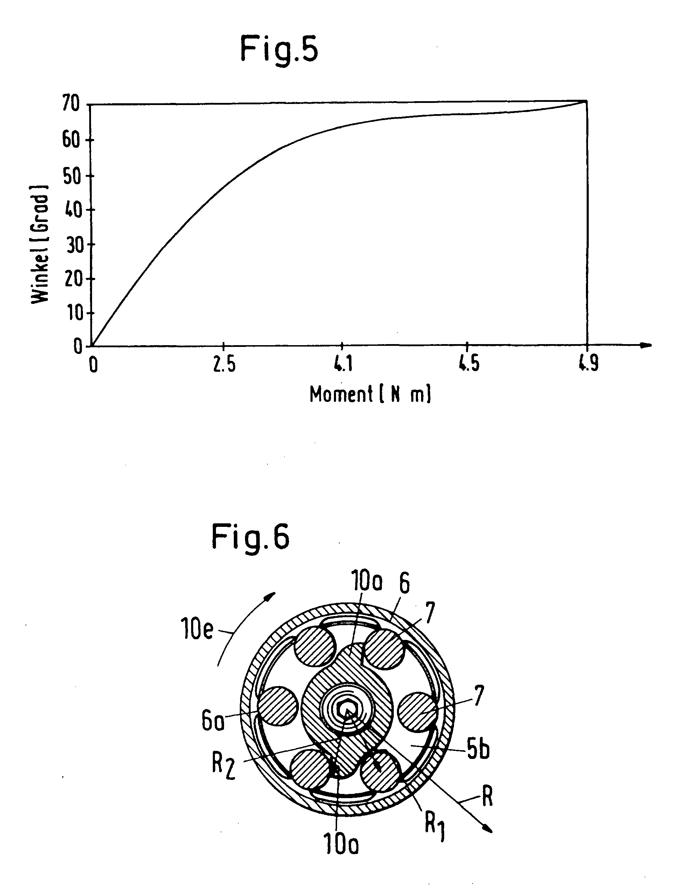

Figur 5 zeigt für ein bestimmtes Material und für eine bestimmte geometrische Ausgestaltung eines Scherkörpers 7 den Zusammenhang zwischen dem in Fig. 3c dargestellten Winkel α der Klinge 10d des Schermittels 10a, sowie das resultierende, maximale Moment. Durch eine entsprechende Wahl des Winkels α kann das maximale Drehmoment relativ genau eingestellt werden. Dazu können in einem Bausatz für das Werkzeug 1 eine Mehrzahl von Wellen 10 mit Klingen 10d unterschiedlichen Winkels α vorrätig sein, sodass die zum Erreichen eines gewünschten maximalen Drehmomentes erforderliche Klinge 10d in die Aufnahmevorrichtung 5 eingesetzt werden kann. Wird ständig ein Werkzeug 1 mit demselben Schermittel 10a bzw. mit demselben Winkel α verwendet, so kann das maximale Drehmoment dadurch variiert werden, dass unterschiedliche Scherkörper 7 zum Einsetzen de die Aufnahmevorrichtung 5 zur Verfügung stehen, wobei diese Scherkörper 7 beispielsweise aus unterschiedlichem Material bestehen, oder eine unterschiedliche Dichte oder eine unterschiedliche Geometrie haben. Weitere Möglichkeiten das maximale Drehmoment zu beeinflussen bestehen, wie in Fig. 6 dargestellt, darin, die radiale Länge R2 des Schermittels 10a zu variieren und/oder gleichzeitig mehrere Schermittel 10a zu verwenden.. Im dargestellten Ausführungsbeispiel ist die radiale Länge R2 geringer als der Abstand R1 des Mittelpunktes des Scherkörpers 7 vom Drehzentrum des Schermittels 10a. Der Scherkörper 7 wird daher vom Schermittel 10a nur teilweise durchtrennt. Schermittel 10a könnten mit einer Klinge 10d, oder wie in Fig. 6 dargestellt mit zwei Klingen 10d, oder beispielsweise mit drei Klingen 10d ausgestaltet sein. Durch eine entsprechende Variation der Anzahl Klingen 10d könnten beispielsweise folgende maximale Drehmomente erzielt werden:

**Tab. 1 Zusammenhang von Anzahl Klingen und maximalem Drehmoment**

| Anzahl Klingen | Maximales Drehmoment | Maximale Anzahl des Gebrauchs bei 6 Abscherstiften |
|---|---|---|
| 1 | 4 Nm | 6 x |
| 2 | 8 Nm | 3 x |
| 3 | 12 Nm | 2 x |

Somit kann durch eine entsprechende Wahl der Anzahl Klingen 10d des Schermittels 10a das maximale Drehmoment variiert werden. Dazu werden vorzugsweise identische Abscherstifte 7 verwendet. Weitere unterschiedliche Werte des maximalen Drehmomentes lassen sich zum Beispiel durch unterschiedlich gestaltete Klingen 10d, oder durch unterschiedlich gestaltete Abscherstifte 7 erzielen.

Die in Fig. 8a dargestellte Welle 10 weist, im Unterschied zu der in Fig. 3a dargestellten Ausführungsform zwei gegenüberliegend angeordnete Schermittel 10a auf. Zudem weist der Endabschnitt des unteren Wellenteils 10c in Umfangsrichtung verteilt angeordnete, rampenförmige Ausnehmungen 10f mit je einer kreisförmigen Vertiefung 10g auf.

Fig. 8b zeigt in einem Längsschnitt ein weiteres Ausführungsbeispiel einer Werkzeughaltevorrichtung. Gleiche Bezugszeichen bezeichnen in den Figuren 1c und 8b dieselben Gegenstände. Im Unterschied zu dem in Fig. 1c dargestellten Schnitt ist der Schnitt in Fig. 8b derart gelegt, dass dieser nicht durch die Abscherstifte 7 verläuft sondern vollständig durch die metallische Aufnahmevorrichtung 5. Die sich üblicherweise in der Aufnahmevorrichtung 5 befindlichen Abscherstifte 7 sind in Fig. 8b nicht dargestellt. Die Welle 10 ist wie in Fig. 8a ausgestaltet. Ein in der Aufnahmevorrichtung 5 federn angeordneter Stift 5f drückt mit dessen Spitze in die rampenförmige Ausnehmung 10f bzw. in die kreisförmige Vertiefung 10g und bewirkt dadurch in der einen Drehrichtung einen Freilauf. Bei einer Drehung des Handgriffs 3 nach rechts erfolgt die Übertragung des Drehmomentes über die Abscherstifte 7 und das Schermittel 10a auf die Welle 10 beziehungsweise auf das mit der Welle 10 verbundene Werkzeug 4. Bei einer Drehung des Handgriffs 3 nach links erfolgt die Übertragung des Drehmomentes über den federnden Stift 5f und die kreisförmige Vertiefung 10g direkt auf die Welle 10 und das damit verbundene Werkzeug 4. Diese Anordnung weist den Vorteil auf, dass beim Anziehen einer Schraube das maximale Drehmoment begrenzt ist, wogegen zum Lösen der Schraube der Stift 5f ein begrenztes Drehmoment vom Handgriff 3 zum Werkzeug 4 zu übertragen erlaubt. Die Grösse des derart maximal übertragbaren Drehmoments kann abhängig von der jeweils gewählten Konstruktion von sehr klein bis sehr gross sein, um insbesondere ein Drehmoment zu erzeugen, welches zum Lösen einer Schraube genügt.

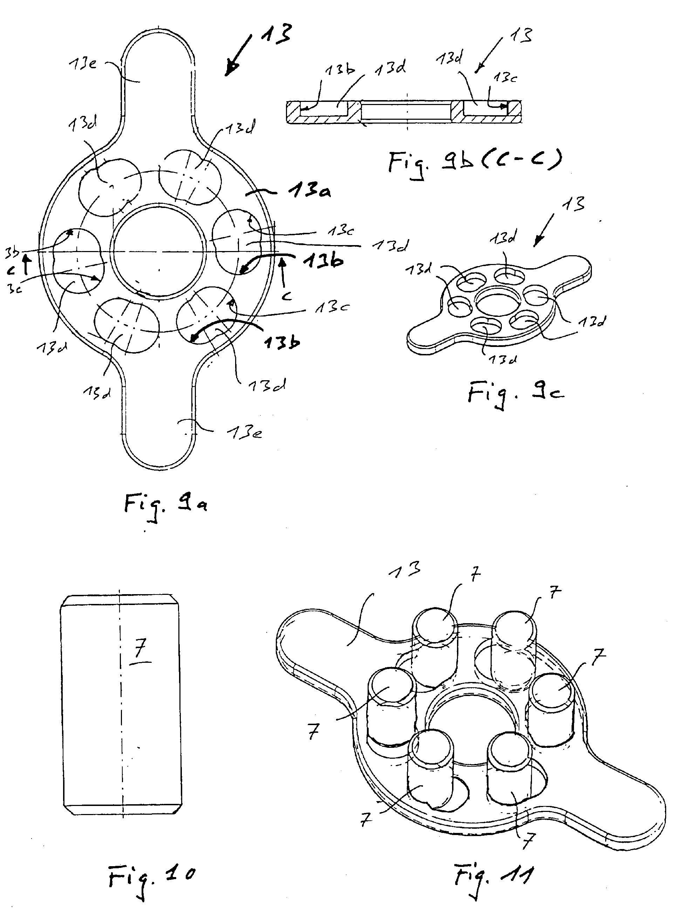

Fig. 9a zeigt eine Aufsicht einer Haltevorrichtung 13 für Abscherstifte 7. Die Haltevorrichtung 13 weist einen kreisförmigen Träger 13a mit zwei Laschen 13e auf, wobei im Träger 13a in Umfangsrichtung gleichmässig beabstandet sechs Ausnehmungen 13d angeordnet sind. Jede Ausnehmung 13d weist eine Bohrung 13b mit einem kleineren Durchmesser, und in Umfangsrichtung versetzt angeordnet eine Bohrung 13c mit einem grösseren Durchmesser auf. Fig. 9b zeigt einen Schnitt durch die Haltevorrichtung 13 entlang der Linie C-C. Fig. 9c zeigt eine perspektivische Ansicht der Haltevorrichtung 13.

Fig. 10 zeigt eine Seitenansicht eines Abscherstiftes 7.

Fig. 11 zeigt eine Haltevorrichtung 13 mit sechs eingesetzten Abscherstiften 7. Zum Befestigen der Abscherstifte 7 in der Haltevorrichtung 13 werden diese vorerst in die Bohrung 13c mit grösserem Durchmesser eingeführt, und dann gegen die Bohrung 13b mit kleinerem Durchmesser hin verschoben, sodass sich ein Klemmsitz ergibt und der Abscherstift 7 dadurch gehalten ist. Um die Abscherstifte 7 in der Aufnahmevorrichtung 5 nachzufüllen werden diese vorerst, wie in Fig. 11 dargestellt, in der Haltevorrichtung 13 angeordnet. Danach werden die derart gehaltenen Abscherstifte 7 gemeinsam in die jeweiligen Halteöffnungen 5c der Aufnahmevorrichtung 5 eingeführt. Danach wird die Haltevorrichtung 13 bezüglich der Aufnahmevorrichtung 5 leicht gedreht, sodass der Klemmsitz gelöst wird, indem die Bohrung 13c mit grösserem Durchmesser die Abscherstifte 7 umgibt. Das Haltemittel 13 kann nun abgehoben werden, wobei die Abscherstifte 7 in den Halteöffnungen 5c verbleiben.

## Patentansprüche

1. Medizinaltechnische Werkzeughaltevorrichtung (2) mit Drehmomentbegrenzung, umfassend eine Aufnahmevorrichtung (5) zum Halten zumindest eines Scherkörpers (7) sowie ein rotierbar mit der Aufnahmevorrichtung (5) verbundenes Schermittel (10a), wobei das Schermittel (10a) und die Aufnahmevorrichtung (5) derart gegenseitig angepasst ausgestaltet sind, dass das Schermittel (10a) an einem gehaltenen Scherkörper (7) anliegt, zumindest wenn ein Drehmoment auf das Schermittel (10a) wirkt, **dadurch gekennzeichnet dass** das Schermittel (10a) zumindest eine Klinge (10d) aufweist, dass die Klinge (10d) derart angeordnet ist, dass sie auf den Scherkörper (7) einwirkt, und dass beim Überschreiten eines bestimmten Drehmomentes das Schermittel (10a) den Scherkörper (7) zumindest teilweise durchtrennt.

2. Medizinaltechnische Werkzeughaltevorrichtung (2) nach Anspruch 1, **dadurch gekennzeichnet, dass** die Aufnahmevorrichtung (5) mit einem Handgriff (3) und das Schermittel (10a) mit einem Werkzeug (4) verbindbar ist, oder dass die Aufnahmevorrichtung (5) mit dem Werkzeug (4) und das Schermittel (10a) mit dem Handgriff (3) verbindbar ist.

3. Medizinaltechnische Werkzeughaltevorrichtung (2) nach einem der vorhergehenden Ansprüche, **dadurch gekennzeichnet, dass** der Scherkörper (7) aus einem thermoplastischen Kunststoff wie Polyethylen, UHMW-Polyethylen oder aus Metall besteht.

4. Medizinaltechnische Werkzeughaltevorrichtung (2) nach einem der vorhergehenden Ansprüche, **dadurch gekennzeichnet, dass** die Aufnahmevorrichtung (5) zur Aufnahme einer Mehrzahl von Scherkörpern (7), insbesondere sechs Scherkörpern (7), ausgestaltet ist.

5. Medizinaltechnische Werkzeughaltevorrichtung (2) nach einem der vorhergehenden Ansprüche, **dadurch gekennzeichnet, dass** die Aufnahmevorrichtung (5) zusammen mit einem abnehmbaren Gehäuseteil (6) einen Hohlraum bildet, innerhalb welchem das Schermittel (10a) und der Scherkörper (7) angeordnet sind.

6. Medizinaltechnische Werkzeughaltevorrichtung (2) nach Anspruch 5, **dadurch gekennzeichnet, dass** das abnehmbare Gehäuseteil (6) auf dessen Innenseite angeordnete Haltemittel (6a) aufweist, welche zum Halten der Scherkörper (7) bestimmt sind.

7. Medizinaltechnische Werkzeughaltevorrichtung (2) nach einem der vorhergehenden Ansprüche, **dadurch gekennzeichnet, dass** das Schermittel (10a) mit einer um eine Drehachse (10f) drehbar gelagerte Welle (10) fest verbunden ist.

8. Medizinaltechnische Werkzeughaltevorrichtung (2) nach Anspruch 7, **dadurch gekennzeichnet, dass** die Klinge (10d) einen bezüglich der Drehachse (10f) um einen Winkel α geneigten Verlauf aufweist, wobei der Wert des Winkels α insbesondere zwischen 0 Grad und 89 Grad liegt.

9. Medizinaltechnische Werkzeughaltevorrichtung (2) nach einem der vorhergehenden Ansprüche, **dadurch gekennzeichnet, dass** die Klinge (10d) eine zumindest teilweise gerundete Schneidfläche aufweist.

10. Medizinaltechnische Werkzeughaltevorrichtung (2) nach Anspruch 8 oder 9 umfassend eine Klinge (10d) deren radial zur Drehachse (10f) verlaufende Länge zwischen 5mm und 20mm beträgt.

11. Medizinaltechnische Werkzeughaltevorrichtung (2) nach einem der vorhergehenden Ansprüche, **dadurch gekennzeichnet, dass** zumindest die Aufnahmevorrichtung (5) sowie der Scherkörper (7), vorzugsweise auch das Werkzeug (4) sowie der Handgriff (3), aus einem sterilisierbaren Material bestehen.

12. Medizinaltechnische Werkzeughaltevorrichtung (2) nach einem der vorhergehenden Ansprüche, **dadurch gekennzeichnet, dass** diese einen Freilauf umfasst, welcher derart ausgebildet ist, dass das Schermittel (10a) in der einen Drehrichtung bezüglich der Aufnahmevorrichtung (5) drehbar gelagert ist, und dass das Schermittel (10a) in der entgegengesetzten Drehrichtung bezüglich der Aufnahmevorrichtung (5) arretiert ist.

13. Medizinaltechnisches Gerät (1) umfassend eine Werkzeughaltevorrichtung (2) nach einem der vorhergehenden Ansprüche.

14. Bausatz für eine medizinaltechnische Werkzeughaltevorrichtung (2) mit Drehmomentbegrenzung nach einem der Ansprüche 1 bis 12, umfassend
- eine Mehrzahl von Aufnahmevorrichtungen (5) zum Halten zumindest eines Scherkörpers (7), insbesondere in unterschiedlichen Abständen vom Drehzentrum,
- eine Mehrzahl von Scherkörpern (7) unterschiedlicher Form und/oder unterschiedlichen Materials und/oder unterschiedlicher Dichte,
- eine Mehrzahl von als Klingen ausgestaltete Schermittel (10a), wobei die Klingen eine unterschiedliche Länge aufweisen und/oder die Klingen (10d) bezüglich der Drehachse (10f) einen unterschiedlich geneigten Winkel α aufweisen, und wobei jedes Schermittel (10a) eine oder mehrere Klingen (10d) aufweist.

15. Bausatz für eine medizinaltechnische Warkzeughattevorrichtung (2) mit Drehmomentbegrenzung nach Anspruch 14, umfassend eine im wesentlichen kreisförmige Haltevorrichtung (13) mit einer Mehrzahl in Umfangsrichtung beabstandet angeordneter Ausnehmungen (13d), wobei der Durchmesser der Ausnehmungen (13d) derart bezüglich den Scherkörpern (7) angepasst ausgestaltet ist, dass die Scherkörper (7) in den Ausnehmungen (13d) befestigbar sind.

16. Bausatz nach Anspruch 15, **dadurch gekennzeichnet, dass** die Ausnehmungen (13d) derart beabstandet auf der Haltevorrichtung (13) angeordnet sind, dass alle gehaltenen Scherkörper (7) gleichzeitig in die Halteöffnungen (5c) der Aufnahmevorrichtung (5) einführbar sind.

17. Bausatz nach Anspruch 15 oder 16, **dadurch gekennzeichnet, dass** die Ausnehmung (13d) zwei kreisförmige, sich überschneidende Bohrungen (13b, 13c) mit unterschiedlichem Durchmesser umfasst, wobei die Zentren der beiden Bohrungen (13b, 13c) in Umfangsrichtung der Haltevorrichtung (13) leicht versetzt angeordnet sind.

## Claims

1. A medical technological tool holder apparatus (2) with torque limitation, comprising a receiver apparatus (5) for holding at least one shear body (7) as well as a shearing means (10a) which is rotatably connected to the receiver apparatus (5), wherein the shearing means (10a) and the receiver apparatus (5) are mutually matched to one another in such a way that the shearing means (10a) contacts a held shear body (7), at least when a torque acts on the shearing means (10a); **characterized in that** the shearing means (10a) has at least one blade (10d); **in that** the blade (10d) is arranged such that it can act on the shear body (7); and **in that** the shearing means (10a) at least partly severs the shear body (7) when a specific torque is exceeded.

2. A medical technological tool holder apparatus (2) in accordance with claim 1, **characterized in that** the receiver apparatus (5) can be connected to a handle (3) and the shearing means (10a) can be connected to a tool (4); or **in that** the receiver apparatus (5) can be connected to the tool (4) and the shearing means (10a) can be connected to the handle (3).

3. A medical technological tool holder apparatus (2) in accordance with one of the preceding claims, **characterized in that** the shear body (7) is composed of a thermoplastic such as polyethylene, UHMW polyethylene or of metal.

4. A medical technological tool holder apparatus (2) in accordance with any one of the preceding claims, **characterized in that** the receiver apparatus (5) is designed for the reception of a plurality of shear bodies (7), in particular of six shear bodies (7).

5. A medical technological tool holder apparatus (2) in accordance with any one of the preceding claims, **characterized in that**, together with a removable housing part (6), the receiver apparatus (5) forms a cavity within which the shearing means (10a) and the shear body (7) are arranged.

6. A medical technological tool holder apparatus (2) in accordance with claim 5, **characterized in that** removable housing part (6) has holding means (6a) which are arranged at its inner side and which are intended for the holding of the shear body (7).

7. A medical technological tool holder apparatus (2) in accordance with any one of the preceding claims, **characterized in that** the shearing means (10a) is fixedly connected to a shaft (10) which is rotatably journalled about an axis of rotation (10f).

8. A medical technological tool holder apparatus (2) in accordance with claim 7, **characterized in that** the blade (10d) has an extent which is inclined with respect to the axis of rotation (10f) by an angle α, with the value of the angle α lying in particular between 0 degrees and 89 degrees.

9. A medical technological tool holder apparatus (2) in accordance with any one of the preceding claims, **characterized in that** the blade (10d) has an at least partly rounded cutting surface.

10. A medical technological tool holder apparatus (2) in accordance with claim 8 or claim 9, comprising a blade (10d), the length of which radially extending with respect to the axis of rotation (10f) amounts to between 5 mm and 20 mm.

11. A medical technological tool holder apparatus (2) in accordance with any one of the preceding claims, **characterized in that** at least the receiver apparatus (5) and the shear body (7), preferably also the tool (4) and the handle (3), consist of a sterilizable metal.

12. A medical technological tool holder apparatus (2) in accordance with any one of the preceding claims, **characterized in that** it comprises a free-wheel mechanism which is formed such that the shearing means (10a) is rotationally stored in the one direction of rotation with respect to the receiver apparatus (5); and **in that** the shearing means (10a) is locked in the opposed direction of rotation with respect to the receiver (5).

13. A medical technological device (1) comprising a tool holder apparatus (2) in accordance with any one of the preceding claims.

14. A kit for a medical technological tool holder apparatus (2) with torque limitation in accordance with any one of the claims 1 to 12, comprising
- a plurality of receiver apparatuses (5) for holding at least one shear body (7), in particular at different distances from the center of rotation,
- a plurality of shear bodies (7) of different shape and/or of different materials and/or of different densities,
- a plurality of shearing means (10a) which are designed as blades, with the blades having a different length and/or the blades (10d) having a differently inclined angle α with respect to the axis of rotation (10f), and with each shearing means (10a) having one or more blades (10d).

15. A kit for a medical technological tool holder apparatus (2) with torque limitation in accordance with claim 14, comprising an essentially circular holder apparatus (13) with a plurality of recesses (1 3d) arranged spaced apart from one another in the direction of the periphery, with the diameter of the recesses (13d) being matched with respect to the shear bodies (7) such that the shear bodies (7) can be fixed in the recesses (13d).

16. A kit in accordance with claim 15, **characterized in that** the recesses (13d) are spaced at the holder apparatus (13) such that all shear bodies (7) held are simultaneously insertable into the holder openings (5c) of the receiver (5).

17. A kit in accordance with claim 15 or claim 16, **characterized in that** the recess (13d) comprises two circular bores (13b, 13c) of differing diameter, with the bores overlapping one another, and with the centers of the two bores (13b, 13c) being arranged slightly offset in the direction of the periphery of the holder apparatus (13).

## Revendications

1. Dispositif porte-outil médical (2) avec limitation de couple, comprenant un dispositif récepteur (5) pour tenir au moins un corps de cisaillement (7) ainsi qu'un organe de cisaillement (10a) capable de rotation et relié au dispositif récepteur (5), dans lequel l'organe de cisaillement (10a) et le dispositif récepteur (5) sont réalisés de façon réciproquement ajustée de telle manière que l'organe de cisaillement (10a) est appliqué contre un corps de cisaillement (7) maintenu au moins quand un couple de rotation agit sur l'organe de cisaillement (10a),
**caractérisé en ce que** l'organe de cisaillement (10a) comprend au moins une lame (10d), **en ce que** la lame (10d) est agencée de telle façon qu'elle agit sur le corps de cisaillement (7), et **en ce qu'**en cas de dépassement d'un couple de rotation déterminé l'organe de cisaillement (10a) coupe au moins partiellement le corps de cisaillement (7).

2. Dispositif porte-outil médical (2) selon la revendication 1, **caractérisé en ce que** le dispositif récepteur (5) est susceptible d'être relié à une poignée (3) et l'organe de cisaillement (10a) est susceptible d'être relié à un outil (4), ou bien **en ce que** le dispositif récepteur (5) est susceptible d'être relié à l'outil (4) et l'organe de cisaillement (10a) est susceptible d'être relié à la poignée (3).

3. Dispositif porte-outil médical (2) selon l'une des revendications précédentes, **caractérisé en ce que** le corps de cisaillement (7) est en une matière thermoplastique comme polyéthylène, polyéthylène à poids moléculaire extrêmement élevé ou métal.

4. Dispositif porte-outil médical (2) selon l'une des revendications précédentes, **caractérisé en ce que** le dispositif récepteur (5) et conçu pour la réception d'une pluralité de corps de cisaillement (7), en particulier six corps de cisaillement (7).

5. Dispositif porte-outil médical (2) selon l'une des revendications précédentes, **caractérisé en ce que** le dispositif récepteur (5) forme conjointement avec une partie de boîtier amovible (6) une cavité à l'intérieur de laquelle sont agencés l'organe de cisaillement (10a) et le corps de cisaillement (7).

6. Dispositif porte-outil médical (2) selon la revendication 5, **caractérisé en ce que** la partie de boîtier amovible (6) comprend des organes de maintien (6a) agencés sur sa face intérieure et qui sont destinés à maintenir les corps de cisaillement (7).

7. Dispositif porte-outil médical (2) selon l'une des revendications précédentes, **caractérisé en ce que** l'organe de cisaillement (10) est fermement relié à un arbre (10) monté en rotation autour d'un axe de rotation (10f).

8. Dispositif porte-outil médical (2) selon la revendication 7, **caractérisé en ce que** la lame (10d) présente un tracé incliné par rapport à l'axe de rotation (10f) d'un angle α, la valeur de l'angle α étant en particulier située entre 0° et 89°.

9. Dispositif porte-outil médical selon (2) selon l'une des revendications précédentes, **caractérisé en ce que** la lame (10d) présente une surface de coupe au moins partiellement arrondie.

10. Dispositif porte-outil médical (2) selon la revendication 8 ou 9, comprenant une lame (10d) dont la longueur orientée radialement par rapport à l'axe de rotation (10f) est entre 5 mm et 20 mm.

11. Dispositif porte-outil médical (2) selon l'une des revendications précédentes, **caractérisé en ce qu'**au moins le dispositif récepteur (5) ainsi que le corps de cisaillement (7), de préférence également l'outil (4) ainsi que la poignée (3), sont en un matériau stérilisable.

12. Dispositif porte-outil médical (2) selon l'une des revendications précédentes, **caractérisé en ce que** celui-ci comprend une roue libre, qui est réalisée de telle façon que l'organe cisaillement (10a) est monté avec possibilité de rotation dans un sens de rotation par rapport au dispositif récepteur (5), et **en ce que** l'organe de cisaillement (10) est arrêté dans le sens de rotation opposé par rapport au dispositif récepteur (5).

13. Appareil médical (1) comprenant un dispositif porte-outil (2) selon l'une des revendications précédentes.

14. Ensemble de composants pour un dispositif porte-outil médical (2) avec limitation de couple, selon l'une des revendications 1 à 12, comprenant
- une pluralité de dispositifs récepteurs (5) pour maintenir au moins un corps de cisaillement (7), en particulier à des distances différentes depuis le centre de rotation,
- une pluralité de corps de cisaillement (7) de formes différentes et/ou de matériaux différents et/ou de densités différentes,
- une pluralité d'organes de cisaillement (10a) conçus sous forme de lames, tels que les lames présentent une longueur différente et/ou les lames (10d) présentent par rapport à l'axe de rotation (10f) un angle α d'inclinaison différent, et dans lequel chaque organe de cisaillement (10a) comprend une ou plusieurs lames (10d).

15. Ensemble de composants pour un dispositif porte-outil médical (2) avec limitation de couple selon la revendication 14, comprenant un dispositif de maintien (13) sensiblement de forme circulaire avec une pluralité d'évidements (13d) agencés à distance en direction périphérique, tels que le diamètre des évidements (13d) est conçu de manière ajustée aux corps de cisaillement (7) de telle manière que les corps de cisaillement (7) peuvent être fixés dans les évidements (13d).

16. Ensemble de composants selon la revendication 15, **caractérisé en ce que** les évidements (13d) sont agencés sur le dispositif de maintien (13) à des distances telles que tous les corps de cisaillement (7) maintenus peuvent être introduits simultanément dans les ouvertures de maintien (5c) du dispositif récepteur (5).

17. Ensemble de composants selon la revendication 15 ou 16, **caractérisé en ce que** l'évidement (13d) comprend deux perçages de forme circulaire (13b, 13c) avec des diamètres différents et qui se recoupent, dans lequel les centres des deux perçages (13b, 13c) sont agencés de façon légèrement décalée en direction périphérique du dispositif de maintien (13).
